Europäisches Patentamt

(19) European Patent Office

Office européen des brevets

(11) Publication number: **0 134 703**
**B1**

(12) **EUROPEAN PATENT SPECIFICATION**

(45) Date of publication of patent specification: **23.03.88**

(21) Application number: **84305302.6**

(22) Date of filing: **03.08.84**

(51) Int. Cl.⁴: **A 61 K 47/00, A 23 K 1/00**

(54) Immunogenic composition comprising live encysted protozoa.

(30) Priority: **04.08.83 GB 8326633**

(43) Date of publication of application:
**20.03.85 Bulletin 85/12**

(45) Publication of the grant of the patent:
**23.03.88 Bulletin 88/12**

(84) Designated Contracting States:
**AT BE CH DE FR IT LI NL SE**

(56) References cited:
**WO-A-81/00576**
**BE-A- 670 837**
**DE-A-2 010 191**
**FR-A-2 350 874**
**FR-A-2 408 351**

(73) Proprietor: **UNILEVER NV**
**Burgemeester s'Jacobplein 1 P.O. Box 760**
**NL-3000 DK Rotterdam (NL)**

(72) Inventor: **Davis, Paul James**
**The Hawthorns Pavenham Road**
**Felmersham Bedfordshire MK43 7EX (GB)**
Inventor: **Harris, Peter**
**22 Orchard Road**
**Raunds Northamptonshire NN9 6DE (GB)**

(74) Representative: **Butler, David John et al**
**Unilever PLC Patents Division P.O. Box 68**
**Unilever House**
**London EC4P 4BQ (GB)**

## Description

The present invention relates to the preparation of edible compositions containing vaccines.

The invention is concerned particularly with orally-administrable immunogenic compositions that contain live protozoa, such as coccidia, that are parasitic in or via the gastrointestinal tract. Such protozoa have as their transmission stage a cyst, i.e. a dormant life-cycle stage having a tough outer coat that protects the parasite from unfavourable conditions outside the body of the host. The parasite emerges from the cyst when ingested by a new host.

It is generally believed that effective oral vaccination against parasitic protozoa can only be achieved effectively if the vaccine comprises the protozoa in the live state. Immunity in the host is established by controlled infection with the parasite. The oral administration of killed parasitic protozoa does not give rise to effective immunity. The live organisms can be attenuated, i.e. their natural virulence characteristics can be modified to render them more suitable for administration as a vaccine, but the need to establish an infection in the host remains essential.

Despite their encysted state, the need to maintain the viability of the protozoa in the vaccine poses problems not normally encountered with traditional vaccines involving simpler organisms, such as bacteria or viruses. The protozoa cannot be freeze-dried, for example, for the purposes of storage. Indeed, encysted protozoa generally are susceptible to dehydration.

The natural excystation of the protozoa following ingestion is geared to the digestive mechanisms in the gut of specific hosts. To establish an infection, the cyst must be disrupted and the enclosed organism released into the gut in a region wherein the organism can develop satisfactorily. Any delay in this natural excystation will tend to result in the protozoa being flushed through the gut by the host's normal digestive functions, and excreted by the host before being able to establish an infection.

The need, on the one hand, to protect the encysted protozoa effectively prior to administration, seems irreconcilable with the need to ensure that the excystation process following ingestion by the host is not significantly hindered. It would be expected that any tough, robust coating which fulfilled the first requirement would render the vaccine ineffective by delaying or preventing excystation.

The present invention provides an improved protozoa vaccine delivery system that helps to maintain the essential viability of the immunogenic organisms without seriously hindering the natural mechanism by which they infect their host following ingestion.

The invention provides an orally administrable immunogenic composition comprising live encysted parasitic protozoa embedded within a firmly set gel matrix from which the protozoa are released following ingestion by a host.

The invention also provides a process for the preparation of such an immunogenic composition, involving the steps of forming a suspension of live encysted parasitic protozoa in gel-forming material, and causing the suspension to set to a firm gel.

The gel matrix should be firm enough to resist handling, i.e. soft or squashy gels will not be sufficiently robust to protect the encysted protozoa from physical damage. The gel matrix can be dried after incorporation of the immunogenic material therein. Indeed, it is a surprising feature of the invention that a gel matrix in dried form can function as an effective vaccine delivery system. Alternatively, the gel matrix can be kept moist until the embedded protozoa are fed to the host, or incorporated in a feedstuff.

In the moist state, the embedded protozoa can be handled in the form of a suspension of gel particles in an aqueous medium, for example.

The use of polysaccharide-based gels is preferred. Preferably a rapidly-setting polysaccharide can be used as the gel base. Preferably the setting is achieved chemically. Low-methoxy pectins can be used, but most preferably the gel is based on an alginate. Mixtures of gel bases can be used.

The use of a polysaccharide gel alone leads to relatively hard matrices on dehydration. This hardness can be advantageous under some circumstances, in increasing the physical protection of the protozoa. Nevertheless, this hardness could be a disadvantage in reducing the ease of digestion of the gel matrix and hence inhibiting the release of the protozoa following ingestion. If desired, the gel matrices can be rendered slightly softer by the incorporation of a humectant or plasticiser together with the polysaccharide. Ideal materials for use in this context are glycerol and liquid paraffin. Preferably such materials should not be present in an amount greater than about 75% by weight of the polysaccharide gel material (expressed on a dry-weight basis).

The physical properties of the gel matrix can be modified by the inclusion of one or more fillers. Such materials can also help to lower the raw material cost of the composition as in general they will be cheaper than pure alginate, for example. The fillers should have a fine particle size, in order that they will not significantly affect the flow of the gel base prior to setting. Preferably the filler should not be a source of metallic ions that might cause premature setting of the gel. This problem can be overcome by the addition of a sequestrant. Fillers such as chalk, skimmed milk powder and whey powder all contain calcium, and would tend to set alginate, for example. Preferred fillers are flours, starches, maltodextrins, dextrins and potato pulp. Inorganic fillers, such as china clay, are also very suitable. Dried poultry manure, suitably milled and sterilised could also be used.

In addition to the encysted protozoa, the gel matrix can incorporate a variety of other ingredients intended to modify the performance of the matrix itself or to render the composition more suitable for oral administration, e.g. as an ingredient in animal feedstuffs. Release of the embedded protozoa during digestion can be facilitated by the use of a mixed gel system or the incorporation of emulsifiers or surfactants in the gel composition. To render the composition more suitable as a feed ingredient it can be beneficial to alter the organoleptic properties of the composition by incorporating materials such as flavours and colouring agents. If desired, the protozoa-containing matrix can also be used as a vehicle in which to incorporate other minor feed ingredients, such as growth promotors and carotenoid pigments.

In one embodiment of the invention, the gel matrix contains live encysted parasitic protozoa and a chemotherapeutic agent effective against the protozoa at an intermediate stage in their life cycle, in an amount sufficient to prevent full development of the protozoa in a host following ingestion. The parasitic protozoa are therefore able to establish themselves briefly in the host following ingestion, so leading to an immune response in the host, but the protozoa are prevented by the chemotherapeutic agent from achieving a full life cycle leading to the release of infective protozoa into the environment. For example, if the protozoa are coccidia, this embodiment will ideally utilise an anticoccidial drug believed to have its peak effect at a late stage in the life cycle of the coccidia, preferably during late schizogony. Examples of appropriate anti-coccidial drugs are nicarbazin, furazolidone, nitrofurazone, nihydrazone, sulphaquinoxalene, sulpanitran, dinsed, ormetroprim, sulphadimethoxine and ethopabate. The administration of a vaccine containing a chemotherapeutic agent prevents transmission of the parasite to other hosts, without allowing drug resistant strains to develop. The presence of the chemotherapeutic agent provides a further safeguard against any possibility that the administration of the infective protozoa might lead to a clinical infection in the host under unusual circumstances. Fully virulent protozoa and/or attenuated protozoa can be used in combination with chemotherapeutic agents, if desired.

Administration of the vaccine of the invention can be achieved in a number of ways, such as:

a) Incorporate in a feedstuff for the host, by blending with nutrient materials during feed manufacture. A pre-mix containing the gel matrix, in particulate form, can be used to facilitate distribution of the protozoa-containing gel particles throughout the bulk of the feedstuff.

b) Simple addition to feed at the same time as the feed is presented to the host, e.g. by sprinkling gel particles onto poultry feed.

c) individual administration to host animals, such as newly-hatched poultry chicks.

The vaccine of the invention can be based on a single strain or species of protozoa, but more preferably broad host-specific immunity should be aimed for by including a selection of common strains or species of protozoa in a single vaccine formulation. The protozoa used can be fully virulent "wild" strains, attenuated strains (e.g. embryo-adapted strains or precocious strains), inter-bred strains (currently available for certain coccidia, such as *Eimeria maxima.*), or strains that have been modified by genetic manipulation. A single vaccine formulation can include a combination of these different strain types, if desired.

The invention will be described further in relation to the control of coccidiosis, but it will be readily apparent to the skilled reader that compositions imparting immunity against other cyst-forming parasitic protozoa can be produced by means of the invention.

The invention can be applied to the control of coccidiosis in any host species that is prone to suffer from the disease. Although long associated with avian species, especially poultry, coccidiosis is now being recognised as important in a much wider range of species. Intensive farming methods have tended to magnify the incidence and seriousness of its outbreaks. Examples of non-avian host animals in which coccidiosis is now recognised as being a problem are pigs, ruminants (especially cattle, sheep and goats) and rabbits. The disease is not usually a serious factor in the health of the adult animal, but can be very damaging to the neonate born into an environment heavily contaminated by the mother.

Coccidiosis has been studied extensively in poultry but has so far received relatively little attention in other species. Accordingly, comprehensive details of the coccidia that infect pigs, for example, are not available although several coccidia have been identified in the literature. For instance, the following coccidia have been observed in pigs: *Eimeria debliecki, E. scabra, E. suis, E. spinosa, E. perminuta, E. neodebliecki, E. porci, E. cerdonis, E. polita* and *Isospora suis.* Specific bovine coccidia that have been identified are: *Eimeria bovis, E. zuernii, E. ellipsoidalis, E. auburnersis, E. cylindrica, E. alabamensis* and *E. bukidnonensis.* Sheep are infected by: *Eimeria minakohlyakimovae, E. ovina, E. intricate* and *E. ahsate.* Goats are infected by *E. arloingi.* Coccidia that have been recorded in rabbits are: *Eimeria intestinalis, E. flavesens, E. magna, E. irresidua, E. periformis, E. Stiedai, E. Perforans, E. neoleporis* and *E. media.*

The invention will, however be particularly described in relation to poultry. In this specification, the term "poultry" is used to denote birds of the order *Galliformes* such as the ordinary domestic fowl or chicken (*Gallus domesticus*), turkeys (*Meleagris*), pheasants (*Phasianus*), partridges (*perdix*), grouse (*Lagopus*), guinea fowl (*Numida*) and peacocks (*Pavo*), and also birds of the order *Anseriformes* such as ducks (*Anas*) and geese (*Anser*).

The domestic fowl (*Gallus domesticus*) can be infected by any of the coccidia *Eimeria tenella, E. necatrix, E. brunetti, E. maxima, E. acervulina* and *E. praecox.* The following coccidia are implicated in infections of turkeys (*Meleagris*): *Eimeria melagrimitis, E. dispersa, E. meleagridis, E. gallopavonis, E. adenoides, E. innocua* and *E. subrotunda.* Domestic ducks (*Anas*) suffer from infection caused by *Tyzzeria perniciosa* and also, it is believed by *Eimeria anatis* which they can acquire from wild ducks (*Anas platyrhyncos*). Geese (*Anser*) can suffer from infections caused by *Eimeria anseris, E. nocens* and *E. parvula*, and in addition it is believed that domestic geese can pick up infections from Canada geese caused

3

by *Eimeria hermani, E. striata* and *E. fulva.* The other poultry species referred to earlier each suffer from infections caused by characteristic coccidia, and the invention is equally appropriate and effective in the control of infections caused by the characteristic coccidia in such other poultry species. All oocyst levels mentioned in this specification should be construed as being per coccidia species present.

Coccidial infections are encountered in all poultry species that are reared by man. Such infections are particularly troublesome when they occur in flocks of birds reared under modern intensive husbandry conditions. Infection can spread rapidly throughout the flock, and at the very least can cause poor growth. Severe infection can lead to death of the birds. Thus for many years considerable effort has been expended in attempts to find reliable prophylactic measures against such infections, and in particular to find ways in which birds can be immunised effectively against the incidence of such infections. The practical benefit of any effective immunising technique will be to promote the growth of poultry to which immunity is imparted, at least in the sense that the negative effects on growth caused by coccidial infections will be counteracted thereby.

It has already been established that poultry which have survived infection by coccidiosis retain some degree of immunity against further infection. In our UK patent specification No. 2 008 404 we describe how poultry can be immunised effectively against coccidiosis if a low level of viable sporulated oocysts of one or more coccidia species infective to the species of poultry concerned is administered in a feedstuff given to the poultry on a continuous or at least frequent basis. In UK patent specification No. 2 008 404 we describe a method for promoting the growth of poultry, in which method the poultry are reared on a diet comprising nutrient feed material containing added viable sporulated oocysts of at least one species of coccidia to which the poultry are susceptible, the oocysts being present in a concentration sufficient only to induce sub-clinical infection in the poultry.

An important embodiment of this earlier invention is a commercial poultry feedstuff comprising nutrient material and containing per kg thereof from about 10 to about 10 000 viable sporulated oocysts of at least one coccidia species to which poultry are susceptible.

By providing the poultry with oocysts in their regular feedstuff, the poultry are able in effect to conduct a controlled progressive self-inoculation. Daily intake of oocysts is dictated by the quantity of feedstuff eaten by each bird, and the level of oocysts in the feedstuff can be regulated by the feedstuff manufacturer whose sophisticated quality control facilities can readily ensure that no risks of overdose can possibly arise. Moreover, by having the oocysts in their normal diet, it is possible to ensure that the chicks are consuming appropriate levels of oocysts as soon as they begin to ingest solid food, and thus the immunising process can begin at a very early moment in their post-hatching life.

In UK patent specification No. 2 008 404 we mention that the oocysts can be seriously dmamaged if they suffer dehydration, and for this reason a feedstuff containing them should never be allowed to dry out completely. Preferably the moisture level in the feedstuff should remain in the range of about 6 to about 12% by weight, although higher moisture levels can be used where this does not lead to spoilage of the feedstuff by the growth of moulds, during storage. The same consideration should apply generally to pre-mixes containing the oocysts from which a poultry feedstuff can be prepared.

The viable sporulated oocysts can be obtained by deliberately infecting host animals such as a donor flock of birds, and collecting the oocysts from their droppings. Techniques for obtaining viable sporulated coccidia oocysts *per se* are well known in the art, and form no part of the present invention. One suitable procedure is described at length in US patent specification No. 3 147 186.

The number of sporulated oocysts incorporated in a composition of the invention will depend on the number required per unit weight of the final feedstuff. Typically, a composition of the invention will include at least 1,000 sporulated oocysts per kg. Preferably this minimum level will be at least about 5,000 per kg, and ideally at least 10,000 per kg. A typical general-purpose composition can contain from about 50,000 to about 500,000 sporulated oocysts per kg. A maximum level is not readily definable: a composition containing in excess of one million sporulated oocysts per kg could be useful where a high oocysts level in the feedstuff is needed.

With regard to the level of oocysts in the final feedstuff, preferably the feedstuff will contain not more than about 2,500 viable sporulated oocysts per kg. Although positive benefits will be obtained by the introduction of the sporulated oocysts into the feedstuff given to an individual bird at any stage of its life, it is preferable that the bird is provided with a feedstuff containing the low levels of sporulated oocysts as soon as it has been hatched and commences feeding. As the bird grows, its daily food requirement increases dramatically. Taking the domestic fowl (*Gallus domesticus*) as an example, at the "first feeding" stage the chick will consume at least about 2 g of feedstuff per day. After 10 days this will have risen to at least about 10 kg per day, and after 30 days the bird will be consuming perhaps 80 g or more per day. The nutritional requirements of the bird change as it develops, and commercially-available poultry feedstuffs are sold with a range of formulations intended for the different stages of the bird's development. Typically such a range will include feedstuffs specifically formulated for "Starters", "Growers" and "Breeders/layers". In order to ensure that at each stage of its development the bird is consuming an appropriate number of sporulated coccidia oocysts per day, it may be appropriate for the different feedstuffs to contain different numbers of oocysts per kg.

For optimum immunization, we believe that a newly-hatched chick should consume about 1 to 20 viable sporulated oocysts per day. During the first 10 days of growth, this level should rise to about 2 to 50 oocysts per day. By day 30, the level of oocysts consumed per day should be of the order of about 5 to 200.

Preferably a "starter" feedstuff will contain at least about 50 viable sporulated oocysts per kg. Preferably the maximum level of oocysts will be about 5,000 per kg, and ideally not more than about 2,000 per kg. In a "grower" feedstuff the minimum level of viable sporulated oocysts is preferably about 50 per kg. The upper level of oocysts is preferably about 2,000 per kg, and ideally not more than about 500 per kg. In a "breeder/layer" feedstuff an appropriate minimum level of viable sporulated oocysts is about 10 per kg. Preferably the upper level of oocysts does not exceed about 1,000 per kg, and ideally is not greater than about 100 per kg.

For host species other than poultry, the daily intake of coccidia oocysts may need to be different depending on factors such as the relative virulence of the organism and the size and susceptibility of the animal involved. In the weaned calf, for example, a daily intake of 1—100 viable sporulated oocysts is probably sufficient.

Certain parasitic protozoa have as their transmission stage "cysts within cysts". Coccidia, for example, exist as sporocysts within an outer cyst (the oocyst). Although for practical purposes it is more convenient to use the whole oocyst as the vaccine ingredient, it will be possible to embed released sporocysts in a firm gel matrix. We therefore contemplate the use of sporocysts in a vaccine in accordance with the invention, although normally oocysts will be preferred.

It will be appreciated that compositions according to the invention can be used also to aid the incorporation of other essential trace ingredients in a feedstuff, for example, mineral additives which are important especially in promoting egg-shell formation in laying birds, vitamins and amino acids such as lysine, and carotenoid pigments commonly used to influence the colour of egg yolks. If desired, one or more of these minor ingredients can be included with the oocysts together in one composite composition.

A composition of the invention can be incorporated in a feedstuff by simple admixture, the proportion of composition per unit weight of the feedstuff being chosen with regard to the concentration of the oocysts present in the composition and the concentration of the oocysts desired in the feedstuff. Apart from the inclusion therein of the composition containing the viable sporulated oocysts, it is not essential for the composition or physical form of a feedstuff to be altered in putting the invention into effect. Any of the currently standard "complete" or nutritionally "balanced" commercial feedstuff formulations can be used, and the nutrient material in the feedstuff can thus comprise any of the protein, carbohydrate and fat ingredients normally found in such feedstuffs. The nature of such standard ingredients, such as the various fish meals, milled grain and other plant material, and mineral additives, and the nutritional requirements such as minimum levels of vitamins, amino acids and trace elements, are well known in the art and documented in the technical literature, and such details form no part of the present invention.

Taking an alginate-based gel as an example of a chemically-settable system, a product according to the invention can be prepared by suspending coccidia oocysts in an aqueous solution of alginate and causing the alginate to gel by means of a suitable chemically initiated reaction. The gelation can be performed in a "bulk setting" system whereby a block of gel is formed which will require subsequent subdivision into a smaller particle size ready for use. Such subdivision can be accomplished by chopping, slicing, or shredding for example. Preferably however, the gelation is accomplished while the product is being prepared in a relatively finely divided form thus rendering subsequent subdivision unnecessary. For example, the setting gel can be extruded through a narrow die thus producing noodle-like structures of relatively small cross-section. The suspension can be exruded into a setting medium.

A preferred process involves contacting droplets of oocyst-containing aqueous alginate solution with a setting medium. The droplets can be generated by spraying, or more preferably by allowing the solution to fall from one or more nozzles. A most preferred technique is to form beadlets of gel by allowing droplets of the oocyst-containing alginate solution to fall into a setting medium, such as a bath containing the setting agent. Under such conditions the droplets generate gel very rapidly and remain as distinct beadlets. After setting, the alginate beadlets can be recovered from the setting medium. If necessary, beadlet size fractions can be selected using standard techniques such as sieving.

A typical size for the oocyst-containing alginate beadlets is about 5 mm diameter when moist, leading to dehydrated beadlets of about 1—3 mm diameter. The final size of the dehydrated beadlets is dependent on the solids content of the beadlets when wet.

The quantity of alginate in the aqueous solution should be sufficient at least to maintain the integrity of the solution when it is contacted with the setting agent. For example, when drops of solution fall into a setting bath, they should gel firmly and immediately, and therefore remain as distinct robust beadlets. Relatively high molecular weight alginates, which lead to firm gels at low concentration, are preferred where substantial quantities of fillers and other components are incorporated in the product. In general, the quantity of alginate in the gel matrix when moist should be from 0.25—10% by weight.

A wide range of commercially available alginates can be used although preferably alginates having a high ratio of mannuronic: guluronic acid residues are preferred. Because the intended use of the vaccine of the invention is in the oral vaccination of animals, it is not necessary for pharmaceutical grade or human food grade alginates to be used, although such materials can be employed if they are economic. The primary requirement is that the level of insoluble matter in the alginate should not prevent formation of a

# 0 134 703

satisfactory product, e.g. by interfering with the flow properties of the alginate solution or reducing the gel strength. Relatively unrefined alginates can be used.

Chemical setting of the alginate can be achieved by use of metallic ions that render the alginate insoluble, particularly calcium. Salts, such as calcium chloride or calcium phosphate, are ideal setting agents. Alternatively, acid setting can be used. Free acids, such as hydrochloric or acetic acid, can be employed.

As an alternative to chemically set gelling systems, it is also possible to use heat-settable gelling materials, particularly proteins. Taking gelatin as an example, a solution of the protein in water should be formed at, for example, about 60°C and the solution cooled, and the coccidia oocysts added at a temperature at which they will remain viable. Gelling of the gelatin will begin at approximately 30°C and this can be facilitated by allowing droplets of the gelatin solution to fall into cold oil.

The concentration of setting agent in the setting bath affects the setting time of the alginate solution. Moreover, if the concentration of setting agent particularly a calcium salt such as calcium chloride is high the salt level in the alginate beadlets may also be high. The beadlets then become deliquescent and may mot dehydrate satisfactorily. Moreover, in the moist beadlets the presence of a high calcium salt concentration exerts a powerful osmotic effect on the oocysts, which should be avoided if possible. In general a maximum level of not more than 5% by weight calcium chloride in the setting bath is required.

However, if the gelled beadlets are left in contact with water for a number of hours, after being left overnight excess calcium chloride will diffuse from the beadlets.

If desired, dehydration of the gel beadlets can be accomplished by air drying and in general a moisture level of less than 20% by weight in the final beadlets should be aimed for.

Preferably however, the gel beadlets are stored in the moist state, for example in contact with an aqueous medium, as this provides the best environment for the oocysts. The beadlets can be incorporated in a feedstuff in the moist state, and allowed to reach an equilibrium moisture level.

The following Examples illustrate embodiments of the invention in greater detail.

Example 1

This example uses a commercially available sodium alginate (Manucol LB) as the gel base. This alginate has a high ratio of mannuronic:guluronic acid residues and a low molecular weight. "Manucol" is a trade name of Kelco/AIL International Ltd.

95 ml of an aqueous solution containing 11% by weight Manucol LB was mixed with 5 ml of an aqueous suspension of viable sporulated coccidia oocysts (*Eimeria tenella*) containing approximately 600 000 oocysts. This mixtures was dropped through a distance of approximately 3 inches (76 mm) into a 5% by weight aqueous solution of calcium chloride dihydrate. The resulting set gel beadlets had an approximate diameter of about 5 mm and a weight of approximately 40 mg. Approximately 2,500 beadlets were obtained during a 2-minute run. The beadlets were allowed to remain in the setting bath for 15 minutes before being sieved clear of the setting agent and then soaked overnight in tap water to remove excess calcium chloride.

The beadlets were allowed to dry on absorbent paper at ambient temperature and there final moisture content was 15—16% by weight.

Example 2

This example uses sodium alginate as the gel material plus glycerol as a humectant.

45 ml of an aqueous solution containing 11% by weight Manucol LB sodium alginate was mixed with 5 ml of a coccidia oocysts suspension (as in Example 1) and 50 ml glycerol. This mixture was subjected to the same beadlet forming procedure as described in Example 1 with the exception that excess calcium chloride was removed from the gelled beadlets by overnight soaking in a 50—50 mixture of water and glycerol. The final moisture content of the dried beadlets was again 15—16% by weight.

The viability of the coccidia oocysts in the alginate beadlets produced according to the two previous examples was demonstrated by the following trial.

After being stored in the dehydrated state for a few more days, a sample of alginate beadlets was blended with 500 grams conventional poultry feed such that each 500 gram sample contained approximately 2,500 beadlets. Each feed sample was fed to two thirty-day old chicks on an *ad lib* basis for two days. All of the birds exhibited clinical symptoms of coccidiosis, with blood in their droppings on the 5th and 6th days following administration of the oocysts-containing feed.

This test indicated that large numbers of the coccidia oocysts remained in viable form despite the processes to which they had been subjected, and yet were still able to excyst satisfactorily in the gut despite being embedded in the firm alginate gel matrix.

Example 3

This experiment was designed to confirm that very low numbers of virulent sporulated oocysts, in a composition in accordance with the invention, when added to poultry feed could survive and cause controlled, sub-clinical infections when fed to susceptible birds. The immunity arising from this treatment, continued for 27 days, was tested by means of a challenge with 50,000 oocysts. Two groups, each of 12 birds, were used. The first group received the oocyst-containing diet from one day old and the second

**0 134 703**

group received a normal, oocyst-free diet. The birds were kept on wire so that the daily oocyst discharge resulting from the oocysts in the diet could easily be monitored. The accommodation in which the birds were housed had been thoroughly treated to ensure freedom from residual oocysts.

Oocysts were obtained by infecting 4-week old Cobb broiler chicks by an oral dose of 50,000 oocysts per bird. Their droppings were collected on days 6, 7 and 8 after infection, mixed with approximately twice their volume of 2.5% aqueous potassium dichromate solution and then homogenised to give a smooth slurry. The slurry was strained through a nylon gauze and the oocysts were recovered from the filtrate by salt floatation, as follows. Firstly, the oocysts were sedimented by centrifugation at 1,000×g for 15 minutes and the sediment was then resuspended in saturated aqueous sodium chloride solution. This was subjected to centrifugation at 500×g for 10 minutes, and the oocysts suspension which remained was drawn off and diluted with 10 volumes of distilled water. Centrifugation at 1,000×g for 15 minutes brought the oocysts down into a pellet. The whole salt floatation procedure was repeated, and the resulting oocysts pellet was finally taken up in 2.5% aqueous potassium dichromate. The extracted oocysts were sporulated by continuous aeration of this suspension for four days at 28°C. As a stock suspension, these oocysts were maintained at 4°C in phosphate buffered saline.

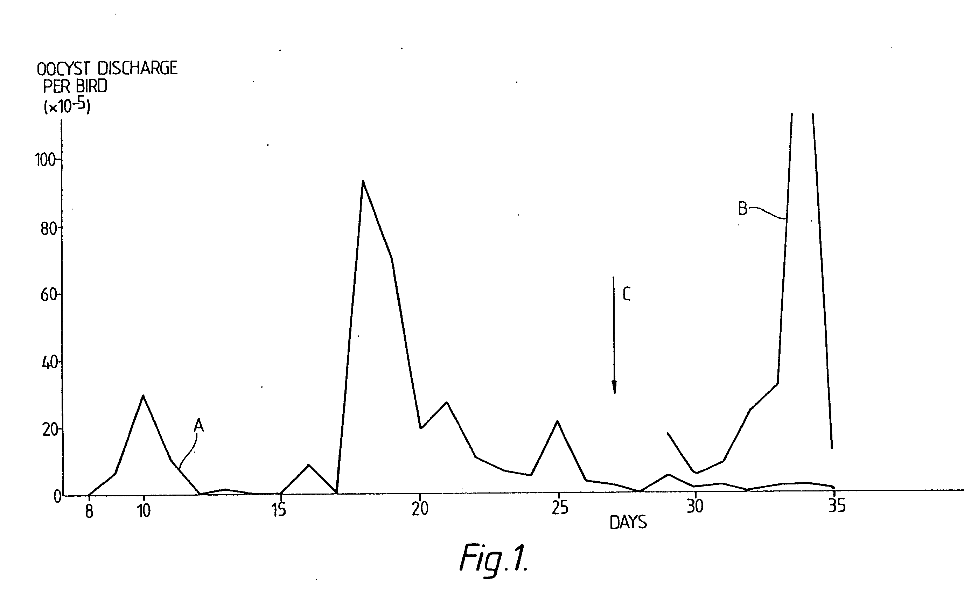

Beadlets were produced as in Example 1, except that a total of 30 000 sporulated coccidia oocysts were used. 240 beadlets were added to 3 Kg of poultry feed and fed to newly-hatch chicks for a period of 3 weeks. The excreted oocyst level from each group was monitored and the results are shown in Figure 1.

The oocysts survived in the feed for the duration of the experiment, as oocysts were passed from day 8 right throughout the immunizing period, as shown in Figure 1. It was obvious from the pattern of oocyst discharge (line A), Figure 1 that a substantial immunity was beginning to develop soon after the birds reached barely 2 weeks of age, for a soaring oocyst discharge was promptly checked and brought under control by day 21.

The immunizing oocysts produced no detectable pathogenic effects. No bloody droppings were seen throughout the whole immunizing period but, more importantly, the mean weight gains of the control group and the immunized group were virtually identical up to the day of challenge.

Challenge (C) with 50,000 oocysts on day 27 caused severe disease in the control group as judged by the presence of blood in the droppings and a profuse discharge of oocysts (line B, Figure 1). The immunized group were apparently completely unaffected but there was no mortality in either group.

Examples 4—7

These examples illustrate the use of fillers in alginate beadlet formation.

Beadlets were prepared following the procedure of Example 1, using the "solution" specified below:

| Example | Alginate | Filler |
|---|---|---|
| 4 | 1% Manucol LM | 10% commercial white wheat flour |
| 5 | 0.25% Manucol LM | 10% commercial white wheat flour |
| 6 | 1% Manucol LM | 10% china clay |
| 7 | 0.25% Manucol LM | 10% china clay |

In each instance, after setting and drying as in Example 1, robust beadlets were obtained.

Manucol LM is an alginate having a high ratio of mannuronic:guluronic acid residues and a high molecular weight. A high MW alginate is preferred when a filler is used, in order to ensure adequate gel strength.

**Claims**

1. An orally administrable immunogenic composition comprising live encysted parasitic protozoa, embedded within a firmly-set gel matrix from which said protozoa are released following ingestion by a host.

2. A composition according to claim 1, containing per kg at least 1,000 lives encysted parasitic protozoa.

3. A composition according to claim 1, in which said gel is a polysaccharide gel.

4. A composition according to claim 3, wherein said polysachharide is alginate.

5. A composition according to claim 1, in wich said gel is a protein gel.

6. A composition according to any one of the preceding claims, in which said protozoa are coccidia in the form of sporulated oocysts or sporocysts.

7. A composition according to any one of the preceding claims, additionally containing a chemotherapeutic agent effective against said protozoa at a late stage in their life cycle, in an amount sufficient to prevent full development of said protozoa in a host following ingestion.

7

8. A composition according to claim 7, in which said protozoa are coccidia and said chemotherapeutic agent is an anti-coccidial drug having its peak effect in a late stage in the life cycle of said coccidia.

9. A composition according to any one of the preceding claims, in the form of gel matrix beadlets.

10. A process of the preparation of a composition according to claim 1, involving the steps of forming a suspension of said live encysted parasitic protozoa in gel-forming material, and causing said suspension to set to a firm gel.

11. A process according to claim 10, in which said gel-forming material is a water-soluble polysaccharide, and gel formation is achieved by rendering said polysaccharide water-insoluble.

12. A process according to claim 11, in which said gel-forming material is a water-soluble alginate, and in which setting of said gel is caused by contacting said suspension with a source of metallic ions which render said alginate insoluble.

13. A process according to claim 12, in which said source of metallic ions is an aqueous solution of a calcium salt.

14. A process according to any one of claims 10 to 13, in which said gel is formed in bulk.

15. A process according to any one of claims 10 to 13, in which setting of said gel is effected by extruding said suspension into a setting medium.

16. A process according to any one of claims 10 to 13, in which setting of said gel is effected by allowing said suspension to fall drop-wise into a setting medium.

17. A process for the preparation of an orally administrable immunogenic composition containing per kg at least 1,000 live encysted protozoa, involving the steps of:

a) forming a suspension of said live encysted parasitic protozoa in an aqueous solution of alginate;

b) allowing said suspension to fall drop-wise into an aqueous solution containing metallic ions that cause immediate chemical setting of said drops; and

c) recovering said gelled drops from said ion solution.

18. An oral composition for poultry, comprising particles of water-insoluble alginate containing embedded therein viable sporulated oocysts of one or more species of coccidia to which poultry are susceptible.

19. A composition according to claim 18, in which said particles additionally incorporate an anti-coccidial drug having its peak effect against said coccidia during late schizogony.

20. A feedstuff for poultry, incorporating an oral composition according to claim 18 or claim 19, the oocysts thereby being present in the feedstuff in a concentration sufficient only to induce a sub-clinical infection in the poultry.

**Patentansprüche**

1. Oral verabreichbare immunogene Zusammensetzung, die lebende enzystierte parasitische Protozoen umfaßt, welche innerhalb einer fest gehärteten Gelmatrix eingebettet sind, aus der die genannten Protozoen nach Einnahme durch einen Wirt freigesetzt werden.

2. Zusammensetzung nach Anspruch 1 mit einem Gehalt von mindestens 1000 lebenden enzystierten parasitischen Protozoen pro kg.

3. Zusammensetzung nach Anspruch 1, in der das genannte Gel ein Polysaccharidgel ist.

4. Zusammensetzung nach Anspruch 3, in der das genannte Gel ein Alginat ist.

5. Zusammensetzung nach Anspruch 1, in der das genannte Gel ein Proteingel ist.

6. Zusammensetzung nach einem der vorhergehenden Ansprüche, in der die genannten Protozoen Kokzidien in Form von sporulierten Oozysten der Sporozysten sind.

7. Zusammensetzung nach einem der vorhergehenden Ansprüche, die außerdem ein chemotherapeutisches Mittel, das gegen die genannten Protozoen in einem späten Stadium ihres Lebenszyklus wirksam ist, in einem ausreichenden Anteil enthält, um die volle Entwicklung der genannten Protozoen in einem Wirt nach Einnahme zu verhindern.

8. Zusammensetzung nach Anspruch 7, in dem die genannten Protozoen Kokzidien sind und das genannte chemotherapeutische Mittel ein Antikokzidienarzneimittel ist, das seine Spitzenwirkung in einem späten Stadium in Lebenszyklus der genannten Kokzidien hat.

9. Zusammensetzung nach einem der vorhergehenden Ansprüche in Form von Gelmatrixkügelchen.

10. Verfahren zur Herstellung einer Zusammensetzung nach Anspruch 1, das die Schritte des Bildens einer Suspension der genannten lebenden enzystierten parasitischen Protozoen in gelbildendem Material und des Bewirkens des Härtens der genannten Suspension zu einem festen Gel umfaßt.

11. Verfahren nach Anspruch 10, in dem das genannte gelbildende Material ein wasserlösliches Polysaccharid ist und die Gelbildung dadurch erzielt wird, daß das genannte Polysaccharid wasserunlöslich gemacht wird.

12. Verfahren nach Anspruch 11, in dem das genannte gelbildende Material ein wasserlösliches Alginat ist und in dem das Härten dieses Gels dadurch bewirkt wird, daß die genannte Suspension mit einer Quelle von Metallionen in Berührung gebracht wird, die das genannte Alginat unlöslich machen.

13. Verfahren nach Anspruch 12, in dem die genannte Quelle von Metallionen eine wässerige Lösung eines Kalziumsalzes ist.

14. Verfahren nach einem der Ansprüche 10 bis 13, in dem das genannte Gel in Masse gebildet wird.

**0 134 703**

15. Verfahren nach einem der Ansprüche 10 bis 13, in dem das Härten des genannten Gels durch Extrudieren der genannten Suspension in ein Härtungsmedium bewirkt wird.

16. Verfahren nach einem der Ansprüche 10 bis 13, in dem das Härten des genannten Gels durch tropfenweises Fallenlassen der genannten Suspension in ein Härtungsmedium bewirkt wird.

17. Verfahren zur Herstellung einer oral verabreichbaren immunogenen Zusammensetzung, die pro kg zumindest 1000 lebende enzystierte Protozoen enthält, umfassend die Schritte des

a) Bildens einer Suspension der genannten lebenden enzystierten parasitischen Protozoen in einer wässerigen Alginatlösung;

b) tropfenweisen Fallenlassens der genannten Suspension in eine wässerige Lösung, die Metallionen enthält, welche das sofortige chemische Härten der genannten Tropfen bewirkt; und

c) Gewinnens der genannten gelierten Tropfen aus der genannten Ionenlösung.

18. Orale Zusammensetzung für Geflügel, die Teilchen von wasserunlöslichem Alginat aufweist, die darin eingebettet lebensfähige sporulierte Oozysten einer oder mehrerer Kokzidienarten, für welche Geflügel anfällig ist, enthalten.

19. Zusammensetzung nach Anspruch 18, worin die genannten Teilchen außerdem ein Antikokzidienarzneimittel enthalten, das seine Spitzenwirkung gegen die genannten Kokzidien während später Schizogonie hat.

20. Futtermittel für Geflügel, dem ein orales Mittel nach Anspruch 18 oder Anspruch 19 einverleibt ist, wodurch die Oozysten im Futtermittel in einer ausreichenden Konzentration vorhanden sind, um im Geflügel nur subklinische Infektion zu induzieren.

## Revendications

1. Composition immunogène pour administration par voie orale comprenant des protozoaires parasitaires vivants enkystés, noyés dans une matrice de gel fermement durcie d'où lesdits protozoaires sont libérés après ingestion par un hôte.

2. Composition selon la revendication 1, qui contient par kg au moins 1000 protozoaires parasitaires vivants enkystés.

3. Composition selon la revendication 1, dans laquelle ledit gel est un gel de polysaccharides.

4. Composition selon la revendication 3, dans laquelle ledit polysaccharide est un alginate.

5. Composition selon la revendication 1, dans laquelle ledit gel est un gel de protéines.

6. Composition selon l'une quelconque des revendications précédentes, dans laquelles lesdits protozoaires sont des coccidia sous forme d'ovocystes sporulés ou de sporocystes.

7. Composition selon l'une quelconque des revendications précédentes, qui contient en outre un agent chimiothérapique efficace contre lesdits protozoaires à un stade tardif de leur cycle de vie, en une quantité suffisante pour empêcher le développement total desdits protozoaires dans un hôte après ingestion.

8. Composition selon la revendication 7, dans laquelle lesdits protozoaires sont des coccidia et ledit agent chimiothérapique est un médicament anti-coccidial dont l'effet de pointe se manifeste à une stade tardif du cycle de la vie desdits coccidia.

9. Composition selon l'une quelconque des revendications précédentes, sous forme de perles de matrice de gel.

10. Procédé de préparation d'une composition selon la revendication 1, consistant à former une suspension desdits protozoaires parasitaires vivants enkystés dans une matière formant un gel et à obliger ladite suspension à durcir en un gel ferme.

11. Procédé selon la revendication 10, dans lequel ladite matière formant un gel est un polysaccharide soluble dans l'eau et on réalise la formation du gel en rendant ledit polysaccharide insoluble dans l'eau.

12. Procédé selon la revendication 11, dans lequel ladite matière formant le gel est un alginate hydrosoluble et dans lequel on effectue le durcissement dudit gel en mettant en contact ladite suspension avec une source d'ions métalliques qui rendent insoluble ledit alginate.

13. Procédé selon la revendication 12, dans lequel ladite source d'ions métalliques est une solution aqueuse d'un sel de calcium.

14. Procédé selon l'une quelconque des revendications 10 à 13, dans lequel ledit gel est formé en masse.

15. Procédé selon l'une quelconque des revendications 10 à 13, dans lequel on effectue le durcissement dudit gel en extrudant ladite suspension dans un milieu de durcissement.

16. Procédé selon l'une quelconque des revendications 10 à 13, dans lequel on effectue le durcissement dudit gel en laissant tomber goutte à goutte cette suspension dans un milieu de durcissement.

17. Procédé de préparation d'une composition immunogène qu'on administre par voie orale, contenant par kg au moins 1000 protozoaires vivants enkystés, procédé qui consiste:

a) à former une suspension desdits protozoaires parasites vivants enkystés dans une solution aqueuse d'alginate;

b) à laisser tomber cette suspension goutte à goutte dans une solution aqueuse contenant des ions métalliques, ce qui provoque un durcissement chimique immédiat desdites gouttes; et

c) à récupérer lesdites gouttes gélifiées de la solution d'ions.

9

18. Composition orale pour volaille comprenant des particules d'un alginate insoluble dans l'eau contenant des ovocystes sporulés viables noyés d'une ou plusieurs espèces de coccidia auxquelles la volaille est susceptible.

19. Composition selon la revendication 18, dans laquelle lesdites particules contiennent également un médicament anti-coccidial dont l'effet de point se manifeste contre les coccidia pendant la schizogonie tardive.

20. Aliment pour volaille contenant une composition orale selon la revendication 18 ou 19, les ovocystes étant ainsi présents dans l'aliment en une concentration seulement suffisante pour provoquer une infection infra-clinique de la volaille.

*Fig.1.*